(19) Europäisches Patentamt
European Patent Office
Office européen des brevets

(11) Publication number: 0 257 602 A1

# (12) EUROPEAN PATENT APPLICATION

(21) Application number: 87112231.3

(22) Date of filing: 22.08.87

(51) Int. Cl.4: C07D 499/00 , A61K 31/43 , //C07D207/26

(30) Priority: 25.08.86 US 900066

(43) Date of publication of application: 02.03.88 Bulletin 88/09

(84) Designated Contracting States: AT BE CH DE ES FR GB GR IT LI LU NL SE

(71) Applicant: SCHERING CORPORATION
2000 Galloping Hill Road
Kenilworth, New Jersey 07033(US)

(72) Inventor: McCombie, Stuart W.
28 Hanford Place
Caldwell New Jersey 07066(US)
Inventor: Tagat, Jayaram R.
133 Boynton Court
Westfield New Jersey 07090(US)

(74) Representative: von Kreisler, Alek, Dipl.-Chem. et al
Deichmannhaus am Hauptbahnhof
D-5000 Köln 1(DE)

## (54) (5R,6S,8R)-6-(1-hydroxyethyl)-2-(3R-pyrolidin-2-one-3-yl-)thiopenem-3-carboxylic acid.

(57) There is disclosed the compound (5R,6S,8R)-6-(1-hydroxyethyl)-2-(3R-pyrrolidin-2-one-3-yl)thiopenem-3-carboxylic acid, and pharmaceutically acceptable salts and esters as well as compositions containing them and methods for their use.

EP 0 257 602 A1

Xerox Copy Centre

## (5R,6S,8R)-6-(1-HYDROXYETHYL)-2-(3R-PYRROLIDIN-2-ONE-3-YL-)THIOPENEM-3-CARBOXYLIC ACID

This invention comprises (5R,6S,8R)-6-(1-hydroxyethyl)-2-(3R-pyrrolidin-2-one-3-yl)thiopenem-3-carboxylic acid and pharmaceutically acceptable salts and esters thereof, which possess potent antibacterial activity.

U.S. Patent No. 4,530,793, and Japanese Disclosure 57-176988 each generically disclose a pyrrolidonethio substituent attached to a penem ring. However, the specific stereoisomers of this invention are not disclosed or contemplated. The compounds of this invention exhibit superior antibacterial activity compared to the prior art compounds.

There currently exists a need for new antibacterial agents since continued extensive use of antibacterials gives rise to resistant strains of pathogens, rendering them less effective. Additionally, in certain clinical instances, the antibacterial compounds currently used manifest clinical toxicity limiting the use of such compounds. This invention addresses these and other needs.

(5R,6S,8R)-6-(1-hydroxyethyl)-2-(3R-pyrrolidin-2-one-3-yl)thiopenem-3-carboxylic acid, pharmaceutically acceptable salts and pharmaceutically acceptable esters thereof possess antibacterial activity against both gram-positive and gram-negative bacteria. In particular, the 3R-pyrrolidin-2-one shows unexpectedly potent antibacterial activity over the 3S-form and over the racemic mixture, such that the 3R-form is useful in oral, parenteral and topical preparations as an antibacterial agent.

The invention also comprises pharmaceutical compositions comprising the inventive compounds in combination with a pharmaceutical carrier, use of the compounds to prepare pharmaceutical compositions useful for treating bacterial infection, and a method for making a pharmaceutical composition by mixing the compounds with a pharmaceutically acceptable carrier.

The compounds described herein are advantageous in that they demonstrate antibacterial activity at lower concentrations than compounds currently in use.

The compounds of this invention exhibit low protein binding and their metabolites have little or no unpleasant odor.

When tested in standardized microbiological assays, the compounds of this invention are active against such gram-positive organisms as Staphlococcus aureus and Bacillus subtilis, and such gram-negative organisms as E. coli and Salmonella, at test levels of 0.016 to 5.66 mcg/ml. Additionally, the subject compounds show activity against organisms which produce beta-lactamases, e.g., penicillinase and cephalosporinase, indicating a resistance to degradation by these enzymes. For instance, the sodium salt of (5R,6S,8R)-6-(1-hydroxyethyl)-2-(3R-pyrrolidin-2-one-3-yl)thiopenem-3-carboxylic acid is active against Staphlococcus aureus at a test level of 0.125 mcg/ml. When tested against E. coli 71120101 (a beta-lactamase producing organism) the compound exhibits activity at 0.125 mcg/ml.

As antibacterial agents, the compounds of this invention may be formulated for oral, parenteral, topical and transdermal use.

For oral administration, the compounds of this invention are typically formulated in the form of tablets, capsules, elixirs, or the like. For parenteral administration, the compounds may be formulated into solutions or suspensions. Typical topical formulations are those such as lotions, creams, ointments, sprays, and formulations adapted for use in mechanical delivery devices, e.g., transdermal.

Typical pharmaceutically acceptable carriers for use in the formulations described above are exemplified by: sugars such as lactose, starches such as corn starch, cellulose and derivatives such as sodium carboxymethyl cellulose, and many other carriers well known in the art. The compositions may also contain preservatives, aerosol propellants and coloring, thickening, suspending, dispensing, emulsifying, wetting, stabilizing and buffering agents.

The dosage of the compounds of this invention is dependent upon a variety of factors, e.g., the age and weight of the individual being treated, the mode of administration, and the type and severity of the bacterial infection being prevented or reduced. Typically, the dosage administered per day will be in the range of from about 1 to 250 mg/kg and preferably from about 5 to 20 mg/kg in divided dosages. Typically, the dosage will be administered in dosage units containing convenient amounts, for example, 125, 250 or 500 mg of active ingredient combined with a suitable physiologically acceptable carrier or diluent.

As used herein, "pharmaceutically acceptable salts" means alkali metal salts such as sodium and potassium salts; alkaline earth metal salts such as calcium, magnesium and aluminum salts; amine salts formed from a wide variety of suitable organic amines, i.e., aliphatic, cycloaliphatic, (cyloaliphatic)aliphatic or araliphatic primary, secondary or tertiary mono-, di-or polyamines, or heterocyclic bases, e.g., salts

derived from triethylamine, 2-hydroxyethylamine, di-(2-hydroxyethyl)amine, tri-(2-hydroxyethyl)amine, 4-aminobenzoic acid-2-diethylaminoethyl ester, 1-ethylpiperidine, bicyclohexylamine, N,N'-dibenzylethylenediamine, pyridine, collidine, quinoline, procaine, dibenzylamine, 1-ephenamine and N-alkylpiperidine.

"Pharmaceutically acceptable esters" means physiologically cleavable esters, i.e., metabolizable esters known in the penicillin, cephalosporin and penem arts to be easily cleaved within the body to the parent acid. Examples of such esters are indanyl, phthalidyl, methoxymethyl, glycyloxymethyl, phenylglycyloxymethyl, thienylglycyloxymethyl, acetoxymethyl and pivaloyloxymethyl.

Preparation of the foregoing salts and esters may be carried out according to conventional procedures for forming salts of beta-lactams such as penicillins, cephalosporins and penems. For example, salts of the compound can be formed by treating it with metal compounds such as alkali metal salts of suitable carboxylic acids, or with ammonia or a suitable organic amine, wherein preferably stoichiometric amounts or only a small-excess of the salt-forming agent is used. The pharmaceutically acceptable esters are preparable in a manner analogous to the preparation of the corresponding esters of penicillins and cephalosporins.

The following processes A) to C) may be employed to produce (5R,6S,8R)-6-(1-hydroxyethyl)-2-(3R-pyrrolidin-2-one-3-yl)thiopenem carboxylic acid, and related salts and esters thereof.

The inventive compounds have the formula

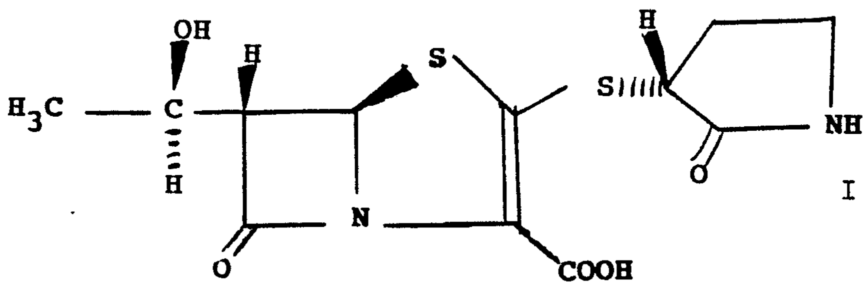

I

and its pharmaceutically acceptable salts and esters.

The processes for producing the compound are characterized by

A) reacting a compound of the general formula

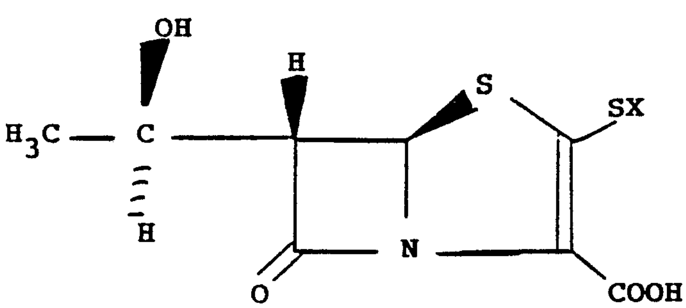

4

wherein X is a alkali metal and the functional groups -OH and -COOH are protected if necessary or desired, with a compound of the formula

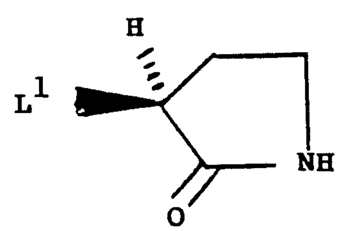

5

wherein $L^1$ is a leaving group, or

B) in presence of base, reacting a compound of the general formula

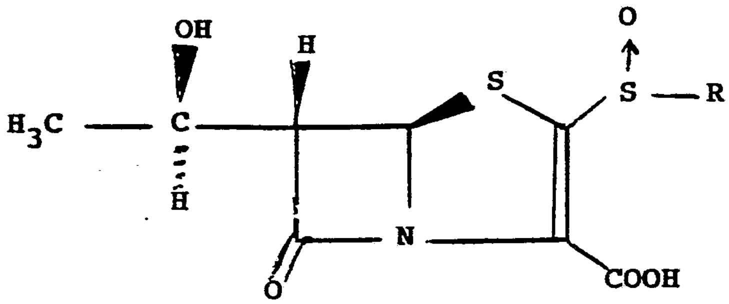

2

wherein R is an organic radical different from the group

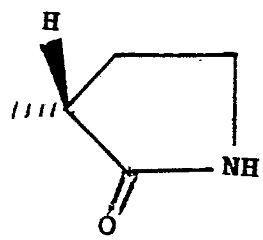

and the functional groups -OH and -COOH are protected if necessary or desired, with a compound of the formula

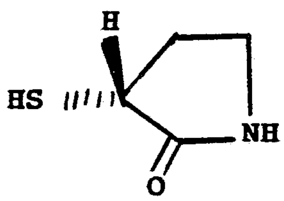

3

3

or,

or,

C) reacting a compound of the general formula

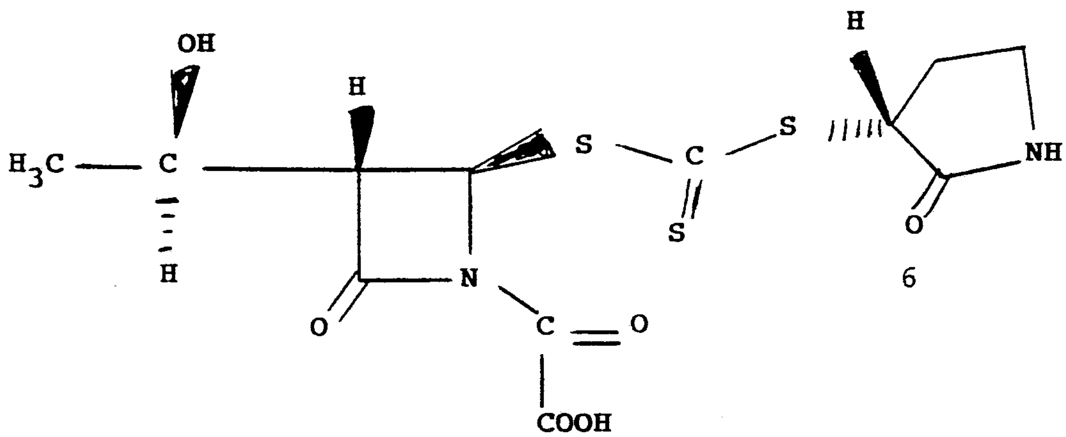

6

in which the -OH and -COOH groups are protected if necessary as desired, with a trivalent organic-phosphorus compound, the processes A), B), or C), being followed by one or more of the following operations, if necessary or desired:

(i) deprotection of one or more protected groups;

(ii) separation of stereoisomers;

(iii) conversion of a free acid to a pharmaceutically acceptable salt or metabolisable ester;

(iv) conversion of a salt or ester to a free acid;

(v) transesterifying an ester or converting an ester into a pharmaceutically accepable salt;

(vi) converting a salt into another salt or an ester.

In process A) the reaction of compounds 4 and 5 is generally carried out in solvent, e.g. an organic solvent, possibly containing water, e.g. acetonitrile and water, or an anhydrous organic solvent, e.g. tetrahydrofuran, ethylether or dioxan. Generally the reaction will be carried out in the presence of a base which may be an organic base, e.g. triethyl amine, or more usually an inorganic base such as sodium bicarbonate. Reaction temperatures are typically from about -10°C to 45°C, with room temperature (about 25°C) being preferred. $L^1$, the leaving group in compound 5, may be chosen from a wide variety of such groups well known to chemists, but preferred $L^1$ groups are $-OSO_2CH_3$, $-OSO_2C_6H_4$-p-$CH_3$, and $-OSO_2CF_3$. The preferred $L^1$ group is $-OSO_2CH_3$.

Compound 5, wherein $L^1$ is $-OSO_2CH_3$ may be made as follows.

The compound (S)-3-hydroxy pyrrolidin-2-one is produced from methyl (S)-4-benzyloxycarbonylamino-2-hydroxybutyric acid by reaction in an acidified inert organic solvent by catalytic reduction.

For example, a small quantity of concentrated hydrochloric acid is used to acidify a methanol reaction medium, and palladium/carbon is introduced under nitrogen to act as a reduction catalyst. After reduction with hydrogen a base such as triethylamine is introduced, and the mixture is heated in an inert solvent such as benzene. The (S)-3-hydroxy pyrrolidin-2-one product is then reacted at about 0°C in an anhydrous inert solvent, e.g. an alkylene dihalide, such as methylene chloride with methane sulfonyl chloride and triethylamine to yield (S)-3-methane sulfonyloxy-pyrrolidin-2-one.

The above lactam mesylate is then subsequently reacted with an alkali metal salt (preferably potassium), of an esterified thiopenem which may be produced in accordance with the disclosure of U.S. Patent No. 4,559,333 to produce the corresponding esterified penem. For example, allyl (5R,6S,8R)-6-(1-hydroxyethyl)-2-potassium-thiopenem-3-carboxylate is reacted with (S)-3-methanesulfonyloxy-pyrrolidin-2-one to form allyl (5R,6S,8R)-6-(1-hydroxyethyl)-2-(3R-pyrrolidin-2-one-3-yl)thiopenem-3-carboxylate.

The allyl group is thereafter easily removed from the carboxyl moiety to form the free carboxylic acid or the sodium carboxylate through reaction with sodium 2-ethylhexanoate or the free acid and a catalyst as disclosed in U.S. Patent 4,314,942. If the free carboxylic acid compound is desired it may be produced through reaction with 2-ethylhexanoic acid to yield (5R,6S,8R)-6-(1-hydroxyethyl)-2-(3R-pyrrolidin-2-one-3-yl)-thiopenem-3-carboxylic acid.

Salts may be alternatively converted in the usual manner into the free carboxy compounds.

Process B) involving sulfoxide replacement is usually carried out in an inert solvent, e.g. dichloromethane or tetrahydrofuran. The reaction is usually carried out under cooling, e.g. -70°C to 10°C, preferably 0°C to 5°C.

Compound 3 can be produced by the following reaction

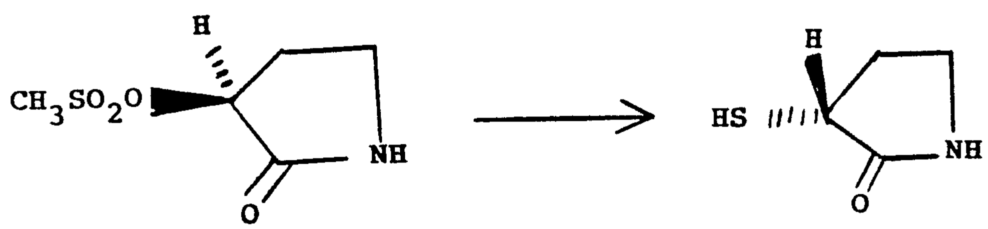

Compound 5 3

wherein $L^1$ is $-OSO_2CH_3$

The reaction takes place in an excess of potassium hydrogen sulfide, in solvent (e.g. ethanol) at about 25°C.

Compound 2 is well known, see, for example, European Patent No. 0 121 502, wherein preparation A prepares a compound of formula 2 wherein R is ethyl. In general R can be a variety of organic radicals.

Process C) involving cyclisation of the compound of formula 6 is usually carried out analogously to the process described in European Patent Application No. 58317. Thus it is usually carried out in an inert solvent for example, an aromatic hydrocarbon, e.g. toluene, benzene, or 1,1,1,2,2-pentachloroethane.

In general the cyclisation reaction is carried out at temperatures in the range of from 20°C to 80°C, usually 40°C to 60°C for about 6 to 24 hours.

Suitable trivalent organophosphorus compounds are cyclic and/or acyclic trialkylphosphites, triarylphosphites and mixed aryl alkylphosphites or phosphoramides. The preferred trivalent organophosphorous compounds are a trialkylphosphites; the most preferred is triethylphosphite.

The compound of formula 6 may be made by the following reaction sequence.

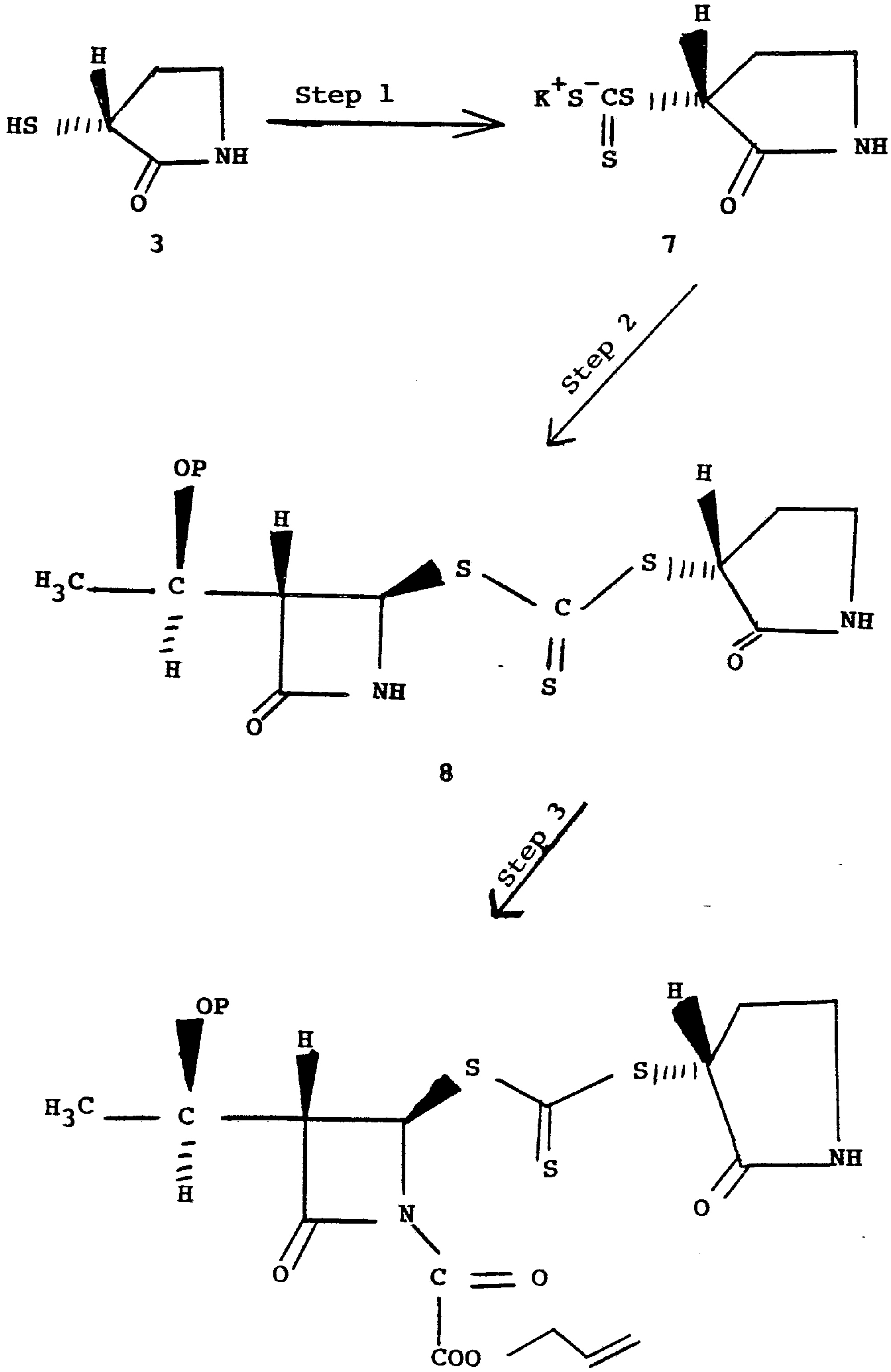


3 7 8

6'

In Step 1 compound 3 is reacted with one equivalent of potssium hydroxide and one or more equivalents of carbon disulfide in ethanol solvent to produce compound 7.

In Step 2 compound 7 is reacted with a compound having the formula

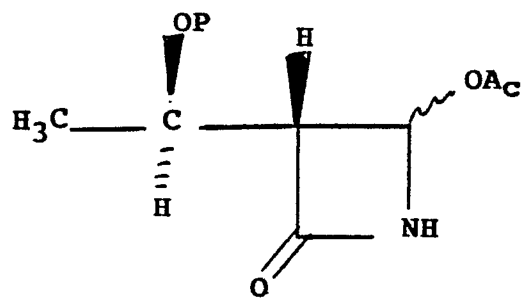

9

wherein p is a hydroxy protecting group, e.g. 2,2,2-trichloroethoxycarbonyl. The reaction takes place at 15-35°C in solvent, e.g. THF. Compound 8 is produced.

In Step 3 compound 8 is reacted with $CH_2=CH-CH_2-OOC-COCl$ in solvent, e.g. $CH_2Cl_2$, in presence of diisopropylethylamine or other hindered tertiary amine. Compound 6′ is formed, which is a protected version of compound 6. In process C, it is preferred that compound 6 be protected.

Generally, in the processes of the invention, the carboxy group of compound 2, 4, and 6 will be protected in the respective process. The hydroxy group of the compounds may also, conveniently, be protected.

Suitable hydroxy protecting groups for use in the process of this invention are such groups conventionally used for such purpose in the β-lactam art and which are readily removable by procedures utilizing elemental zinc or any other conventional procedures. Preferred hydroxy protecting groups are trichloroethyloxycarbonyl, dimethyltributylsilyl, trimethylsilyl oxycarbonyl and trimethylsily; trimethylsilyl is a particularly preferred protecting group and is removable by treatment with a mild aqueous acid, such as aqueous acetic acid.

Suitable carboxy protecting groups are those conventionally used in the penem art and which can be removed under conventional conditions without reaction with other functional groups present on the penem molecule, for example allylic, p-nitrobenzyl and trichloroethyl. The preferred carboxy protecting group is allyl.

The removal of the protecting group from a protected carboxyl group can be carried out by conventional procedures selected according to the identity of the protecting group. For the removal of the preferred protecting group, allyl, this can in general be effected under catalytic conditions in the presence of a base, preferably by utilizing procedures described in our European Patent Application Publication No. 0013663. Thus an allyl group is preferably removed by utilizing a suitable aprotic solvent, such as tetrahydrofuran, diethyl ether or methylene chloride, with an alkali metal alkylcarboxylate, preferably potassium or sodium 2-ethylhexanoate (to give the alkali metal penem salt, preferably the sodium or potassium penem salt directly) or carboxylic acid, preferably 2-ethylhexanoic acid (to give the penem free-acid) and a mixture of a palladium compound and triphenyl phosphine as a catalyst.

The following examples illustrate the preparation of the compounds and compositions of this invention. Unless otherwise specified all temperatures are in degrees Celsius.

EXAMPLE 1

(S)-3-HYDROXY-PYRROLIDIN-2-ONE

Stir at room temperature a solution of (S)-4-benzyloxycarbonylamino-2-hydroxy butyric acid (25.3 g, 100 mmol.) and concentrated HCl (2.0 ml.) in methanol (150 ml.) for 24 hours. Transfer to a Parr bottle and add palladium on carbon (10%, 2.5 g.) under a nitrogen atmosphere. Place the contents under a pressurized hydrogen atmosphere (50 psi) and shake (6 hrs.). Remove the palladium on carbon by filtering through celite. Concentrate the filtrate *in vacuo* to yield a waxy residue, and dissolve in benzene to form a basic solution. Treat the solution with dry triethylamine (3 ml.), raise to reflux temperature, and stir (4 hrs.). Remove the benzene with a rotary evaporator, and subject the residue to flash chromatography on silica gel (25-50% acetone in dichloromethane) to yield the title compound as a white solid.

200 MHz NMR ($CDCl_3$): δ2.05(m,1H), 2.50(m,1H), 3.35(m,2H), 4.30(t,J=8Hz,1H), 4.65(br-S,1H-OH) and 7.15-(br-S,1H-NH).
M.P.: 75-77°C.

EXAMPLE 2

(S)-3-METHANESULFONYLOXY-PYRROLIDIN-2-ONE

Suspend the product of Example 1 (8 g., 79.2 mmol.) in dry methylene chloride (30 ml.) and cool to 0°C. Treat the suspension sequentially with dry triethylamine (16 ml., 118.8 mmol.) and methane sulfonyl chloride (6.74 ml., 87.12 mmol.). Stir at 5-10°C. for 1.5 hours, and subject the mixture to flash chromatography using acetone (5-10%) in dichloromethane to yield the title compound as a white solid.
Optical rotation: $[\alpha]_D^{26}$ = -68.3° (C=0.62, $CHCl_3$) M.P.: 122-124°C. 200 MHz NMR($CDCl_3$): δ2.45(m,2H), 3.20(s,3H), 3.35(m,2H), 5.10(t,J=8Hz,1H) and 7.75(br-S,1H-NH).
MS: 180(M+1).

EXAMPLE 3

ALLYL (5R,6S,8R)-6-(1-HYDROXYETHYL)-2-(3R-PYRROLIDIN-2-ONE-3-YL)THIOPENEM-3-CARBOXYLATE

Dissolve the compound of Example 2 (12 g., 67 mmol.) in dimethyl formamide (50 ml.) and treat with allyl (5R,6S ,8R)-6-(1-hydroxyethyl)-2-potassiumthiopenem-3-carboxylate. Stir the solution at room temperature for 12 hours. Dilute the reaction mixture with dichloromethane (300 ml.) and wash sequentially with aqueous copper sulfate (5%, 50 ml.), water (50 ml., twice), brine, and dry.

Back extract the combined aqueous layers with chloroform (200 ml.) containing ethyl acetate (10%). Combine all organic layers and concentrate in vacuo to a solid. Recrystallize this solid with methylene chloride or by flash chromatography to yield the title compound as a yellow solid.
200 MHz NMR (DMSO-$d_6$): δ1.2(d,3H); 2.1(m,1H); 2.7(m,1H); 3.3(t,J=5Hz,2H); 3.85 (d,J=4Hz,1H); 4.1-(m,2H); 4.7(dd,2H); 5.2(d,J=4Hz,2H); 5.45(d, J=12Hz,1H); 5.75(s,1Hz); 5.95(m,1H); 8.1(br-S; 1H-NH).

EXAMPLE 4

SODIUM (5R,6S,8R)-6-(1-HYDROXYETHYL)-2-(3R-PYRROLIDIN-2-ONE-3-YL)THIOPENEM-3-CARBOXYLATE

The compound of Example 3 (10 g., 27.2 mmol.) is suspended in methylene chloride (1 l.) containing acetone (10%) and stirred vigorously (1 hr.). Add to the solution sequentially tri isopropyl phosphite (3.35 ml., 13.6 mmol.), sodium 2-ethylhexanoate (4.5 g., 27.2 mmol.) and a catalytic amount of palladium acetate (0.61 g., 2.72 mmol.). Stir (2 hrs.) and remove the solvent with a rotary evaporator to obtain a residue. Dissolve the residue in water (400 ml.) and wash with ethyl acetate (100 ml., 3 times). Pass the aqueous extract through a silica gel pad (3 inch, reverse phase), and wash with water (50 ml.) Combine the aqueous filtrates and lyophilize to yield the title compound as a yellow solid.

200 MHz NMR($D_2O$): δ1.2(d,3H); 2.1(m,1H); 2.70(m,1H); 3.3(t, J=5Hz,2H); 3.85(d,J=4Hz,1H); 4.15(m,2H) and 5.60(s,1H).

In the following examples, the term "Active ingredient" is used to designate the compound (5R, 6S, 8R)-6-(1-hydroxyethyl)-2-(3R-pyrrolidin-2-one-3-yl)thiopenem-3-carboxylic acid and an equivalent amount of pharmaceutically acceptable salts or esters thereof.

EXAMPLE 5

Capsules

| No. | Ingredient | mg/capsule | mg/capsule |
|---|---|---|---|
| 1. | Active ingredient | 250 | 500 |
| 2. | Lactose USP | 100 | 50 |
| 3. | Corn Starch, Food Grade | 50 | 43.5 |
| 4. | Microcrystalline Cellulose NF | 95 | 50 |
| 5. | Magnesium Stearate NF | 5 | 6.5 |
| | Total | 500 | 650 |

Method of Manufacture

Mix Items Nos. 1, 2, 3 and 4 in a suitable mixer for 10-15 minutes. Add Item No. 5 and mix for 1-3 minutes. Fill the mixture into suitable two-piece hard gelatin capsules using encapsulating machine.

EXAMPLE 6

Tablets

| No. | Ingredient | mg/capsule | mg/capsule |
|---|---|---|---|
| 1. | Active ingredient | 250 | 500 |
| 2. | Lactose USP | 57 | 114 |
| 3. | Corn Starch, Food Grade, as a 10% paste in Purified Water | 20 | 40 |
| 4. | Corn Starch, Food Grade | 18 | 39 |
| 5. | Magnesium Stearate NF | 5 | 7 |
| | Total | 350 | 700 |

Method of Manufacture

Mix Items Nos. 1 and 2 in a suitable mixer for 10-15 minutes. Granulate the mixture with Item No. 3. Paste wet granulation through a coarse screen (e.g., 1/4") if needed, and dry the wet granules. Mill the dried granules. Combine Item No. 4 and the dried granules and mix for 10-15 minutes. Add Item No. 5 and mix for 1-3 minutes. Compress the mixture to appropriate size and weight on a suitable tablet machine.

EXAMPLE 7

Injectable Powder: (per vial)

| | g/vial | g/vial |
|---|---|---|
| Active Ingredient | 0.5 | 1.0 |

Add sterile water for injection or bacteriostatic water for injection for reconstitution.

EXAMPLE 8

Injectable Solution

| Ingredient | mg/ml | mg/ml |
|---|---|---|
| Active Ingredient | 100 | 500 |
| Methylparaben | 1.8 | 1.8 |
| Propylparaben | 0.2 | 0.2 |
| Sodium Bisulfite | 3.2 | 3.2 |
| Disodium Edetate | 0.1 | 0.1 |
| Sodium Sulfate | 2.6 | 2.6 |
| Water for Injection q.s. ad | 1.0 ml | 1.0 ml |

Method of Manufacture

1. Dissolve parabens in a portion (85% of the final volume) of the water for injection at 65-70°C.
2. Cool to 25.35°C. Charge and dissolve the sodium bisulfite, disodium edetate and sodium sulfate.
3. Charge and dissolve the active ingredient.
4. Bring the solution to final volume by adding water for injection.
5. Filter the solution through 0.22 membrane and fill into appropriate containers.
6. Terminally sterilize the units by autoclaving.

EXAMPLE 9

Injectable Powder: (per vial)

| | g/vial |
|---|---|
| Active Ingredient | 1.0 |
| Sodium Citrate | 1.05 |

pH is adjusted to 6.2 using 0.1N citric acid solution.
Add sterile water for injection or bacteriostatic water for injection for reconstitution.

## Claims

1. (5R,6S,8R)-6-(1-hydroxyethyl)-2-(3R-pyrrolidin-2-one-3-yl)thiopenem-3-carboxylic acid, the pharmaceutically acceptable salts and pharmaceutically acceptable esters thereof.

2. A compound of claim 1 which is an alkali metal salt.

3. A compound of claim 2 wherein the alkali metal is sodium.

4. A compound of claim 1 which is an alkaline earth metal salt.

5. A compound of claim 1 which is an amine salt.

6. A pharmaceutical composition which comprises a compound as defined in any one of claims 1 to 5 in combination with a pharmaceutically acceptable carrier.

7. The use of a compound as defined in any one of claims 1 to 5 for the preparation of a pharmaceutical composition useful for treating bacterial infection.

8. A method for making a pharmaceutical composition comprising mixing a compound as defined in any one of claims 1 to 5 with a pharmaceutically acceptable carrier.

9. A process for preparing a compound as defined by claim 1 characterized by:

A) reacting a compound of the general formula

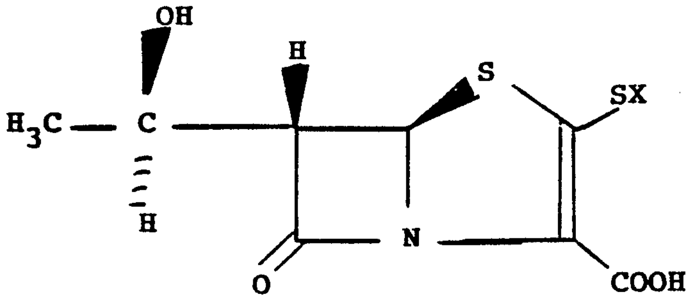

4

wherein X is a alkali metal and the functional groups -OH and -COOH are protected if necessary or desired with a compound of the formula

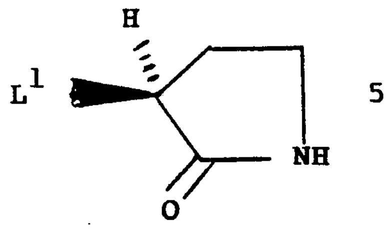

5

wherein $L^1$ is a leaving group, or

B) in presence of base, reacting a compound of the general formula

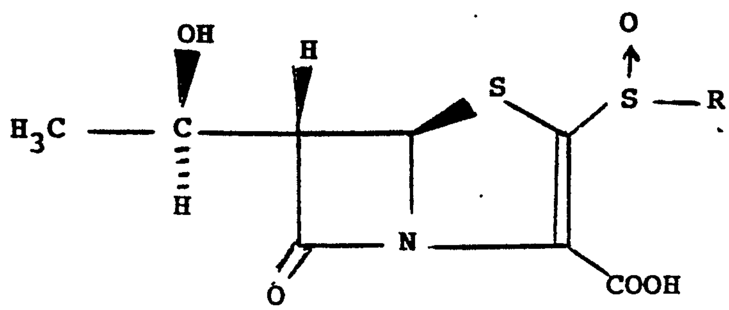

2

wherein R is an organic radical different from the group

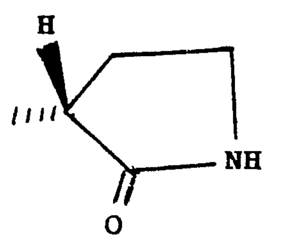 ,

and the functional groups -OH and -COOH are protected if necessary or desired, with a compound of the formula

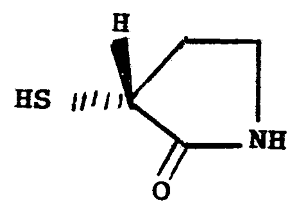

3

C) reacting a compound of the general formula

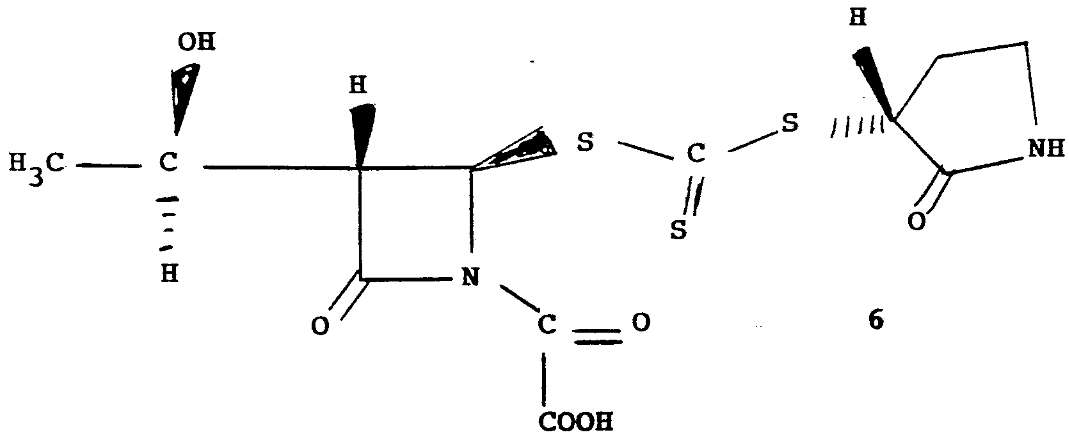

6

in which the -OH and -COOH groups are protected if necessary as desired, with a trivalent organic-phosphorus compound,

the processes A), B), or C), being followed by one or more of the following operations, if necessary or desired:

(i) deprotection of one or more protected groups;
(ii) separation of stereoisomers;
(iii) conversion of a free acid to a pharmaceutically acceptable salt or metabolisable ester;
(iv) conversion of a salt or ester to a free acid;
(v) transesterifying an ester or converting an ester into a pharmaceutically accepable salt;
(vi) converting a salt into another salt or an ester.

Claims for the following Contracting States : AT; GR; and ES

1. A process for preparing (5R,6S,8R)-6-(1-hdyroxyethyl)-2-(3R-pyrrolidin-2-one-3-yl)thiopenem-3-carboxylic acid, represented by the formula

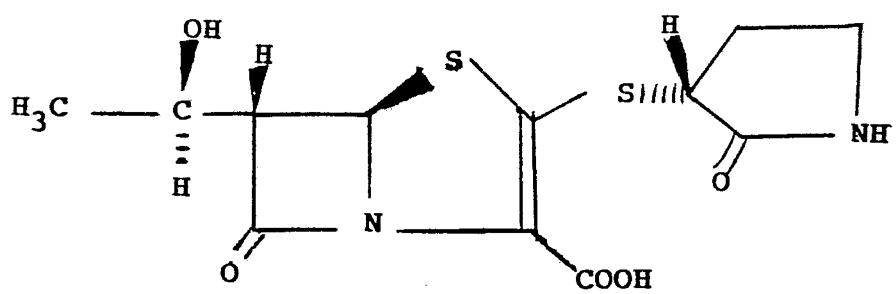

I

and its pharmaceutically acceptable salts and esters characterized by:

A) reacting a compound of the general formula

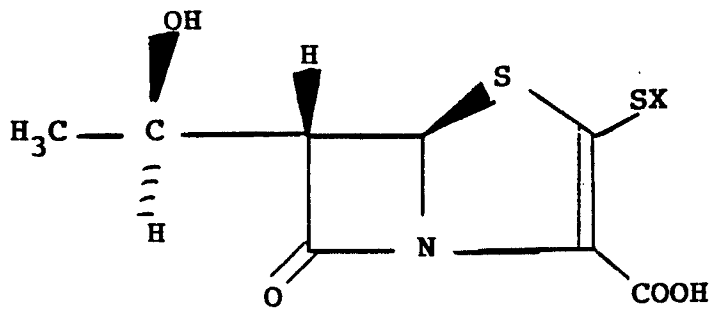

4

wherein X is a alkali metal and the functional groups -OH and -COOH are protected if necessary or desired with a compound of the formula

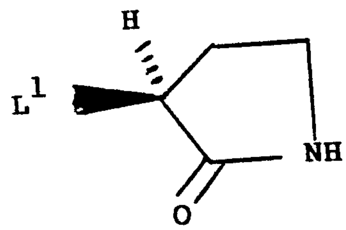

5

wherein $L^1$ is a leaving group, or

B) in presence of base, reacting a compound of the general formula

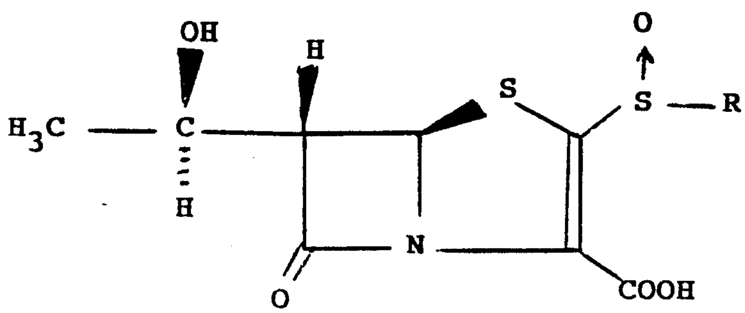

2

wherein R is an organic radical different from the group

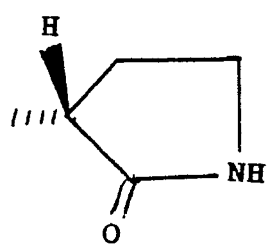

,

and the functional groups -OH and -COOH are protected if necessary or desired, with a compound of the formula

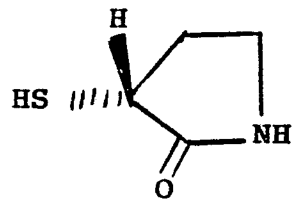

3

C) reacting a compound of the general formula

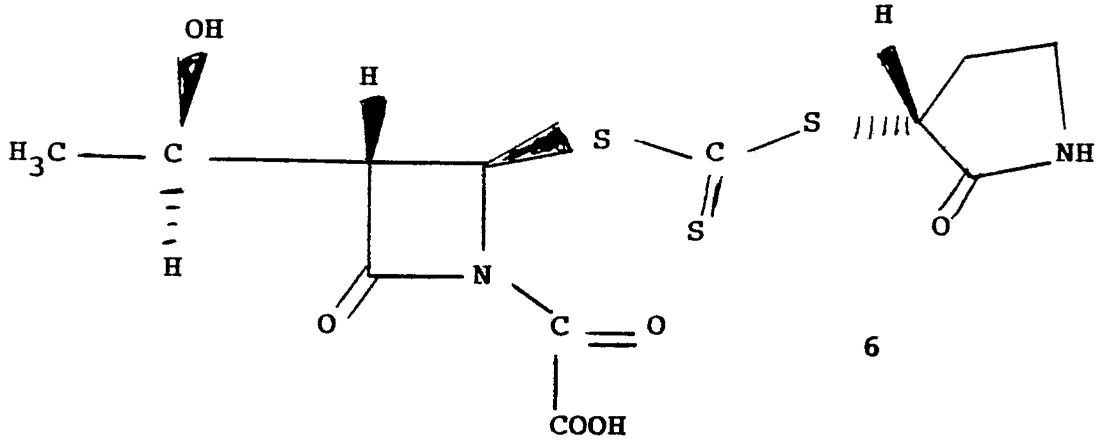

6

in which the -OH and -COOH groups are protected if necessary as desired, with a trivalent organic-phosphorus compound,

The processes A), B), or C), being followed by one or more of the following operations, if necessary or desired:

(i) deprotection of one or more protected groups;
(ii) separation of stereoisomers;
(iii) conversion of a free acid to a pharmaceutically acceptable salt or metabolisable ester;
(iv) conversion of a salt or ester to a free acid;
(v) transesterifying an ester or converting an ester into a pharmaceutically accepable salt;
(vi) converting a salt into another salt or an ester.

2. The process of claim 1 wherein the compound produced is an alkali metal salt.

3. The process of claim 2 wherein the alkali metal is sodium.

4. The process of claim 1 wherein the compound produced is an alkaline earth metal salt.

5. The process of claim 1 wherein the compound produced is an amine salt.

6. A pharmaceutical composition which comprises a compound produced by any one of claims 1 to 5 in combination with a pharmaceutically acceptable carrier.

7. The use of a compound produced by any one of claims 1 to 5 for the preparation of a pharmaceutical composition useful for treating bacterial infection.

8. A method for making a pharmaceutical composition comprising mixing a compound produced by any one of claims 1 to 5 with a pharmaceutically acceptable carrier.

European Patent Office

# EUROPEAN SEARCH REPORT

Application Number

EP 87 11 2231

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | EP-A-0 110 280 (SCHERING) * Page 79, formula; page 96, example no. 91; claims * & US-A-4 530 793 (Cat. D) | 1-9 | C 07 D 499/00<br>A 61 K 31/43 //<br>C 07 D 207/26 |
| Y | EP-A-0 138 539 (PFIZER) * Page 19, preparation A; pages 24,25, preparation D; claims * | 1-9 | |
| | | | **TECHNICAL FIELDS SEARCHED (Int. Cl.4)** |
| | | | C 07 D 499/00<br>A 61 K 31/00 |

The present search report has been drawn up for all claims

| Place of search | Date of completion of the search | Examiner |
|---|---|---|
| THE HAGUE | 24-11-1987 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)